# NEUE EUROPÄISCHE PATENTSCHRIFT

(11) **EP 0 992 490 B2**
(45) Veröffentlichungstag und Bekanntmachung des Hinweises auf die Entscheidung über den Einspruch: **29.08.2007**
(45) Hinweis auf die Patenterteilung: 28.05.2003
(21) Anmeldenummer: 99119279.0
(22) Anmeldetag: 28.09.1999
(51) Int. Cl.: C07C 323/58, A23K 1/00, A23K 1/16

(54) **Rieselfähige Methionin-haltige Formlinge und Verfahren zu deren Herstellung**
Flowable formed methionine products and method for producing the same
Produits de méthionine formés et coulants, et leur procédé de fabrication

(30) Priorität: 10.10.1998 DE 19846825
(43) Veröffentlichungstag der Anmeldung: 12.04.2000
(73) Patentinhaber: Degussa GmbH, 40474 Düsseldorf (DE)
(72) Erfinder: Körfer, Martin, 63867 Johannesberg (DE); Hornung, Gundolf, 63457 Hanau (DE); Huthmacher, Klaus, Dr., 63571 Gelnhausen (DE); Hasselbach, Hans Joachim, Dr., 63571 Gelnhausen (DE); Bönig, Klaus, 63517 Rodenbach (DE)

(56) Entgegenhaltungen:
- EP-A- 0 465 338
- EP-A- 0 959 068
- WO-A-98/37772
- FR-A- 2 032 788
- FR-A- 2 656 772
- US-A- 5 279 832
- DATABASE WPI Section Ch, Week 199241 Derwent Publications Ltd., London, GB; Class B05, AN 1992-337741 XP002069061 -& JP 04 244056 A (SUMITOMO CHEM CO LTD), 1. September 1992 (1992-09-01)
- PATENT ABSTRACTS OF JAPAN vol. 009, no. 066 (C-271), 26. März 1985 (1985-03-26) -& JP 59 199670 A (NIPPON SODA KK), 12. November 1984 (1984-11-12)
- Kopie des Verkaufsprospekts fuer SMARTAMINE vertrieben durch RHONE-POULENC (1993)
- Kopie des Verkaufsprospekts fuer Produkte, die durch RHONE-POULENC ANIMAL NUTRITION 1996 vertrieben worden sind, wobei eine Seite das Produkt SMARTAMINE betrifft
- Internes Dokument von RHONE-POULENC vom 27.10.92
- Zwei Verkaufsrechnungen von SMARTAMINE vom 26.02.97 und vom 27.02.97
- Internes Dokument der AVENTIS ANIMAL NUTRITION
- PATENT ABSTRACTS OF JAPAN vol. 009, no. 066 (C-271), 26. Maerz 1985 (1985-03-26) -& JP 59 199670 A (NIPPON SODA KK), 12. November 1984 (1984-11-12)

## Beschreibung

Die Erfindung betrifft rieselfähige Methionin-haltige Formlinge und ein Verfahren zur Herstellung dieser Produkte.

Die Herstellung von Methionin oder einem Salz des Methionins, dieses insbesondere als Zwischenstufe vor der Isolierung des Methionins in einem entsprechenden Verfahren, ausgehend von den Ausgangsstoffen Methylmercaptopropionaldehyd und Cyanwasserstoff ist seit langem bekannt und in einer Vielzahl von Literaturstellen beschrieben.
Über die Zwischenstufe des 5-(β-mercaptoethyl)-Hydantoins und die sich anschließende basische Hydrolyse mit unterschiedlichen Alkali-Erdalkali- oder Ammoniumverbindungen wird dabei das entsprechende Methioninsalz erzeugt. Aus diesem setzt man mit Hilfe von Säuren Methionin frei und isoliert dies durch ein geeignetes Verfahren. Die Aufarbeitungsverfahren zur Überführung des Methionins in einen zur weiteren Verwendung geeigneten Zustand erweisen sich im allgemeinen als sehr aufwendig.

Das nach bekannten Verfahren (US-PS 2,557,913, DE-PS 1906405, JP 42/44056) erzeugte-Methionin zeichnet sich durch eine mehr oder weniger stark ausgeprägte kristalline Struktur aus. Es liegt als weißes bis cremig-weißes Produkt vor; die Schüttgewichte liegen im allgemeinen zwischen 600 und 700 kg/m³, der Kornspektrumbereich ist sehr weit gestreut zwischen <32 µm und ca. 1200 µm, die Rieselfähigkeit kann insbesondere bei Vorliegen der Plättchenstruktur des Methionins stark beeinträchtigt sein.

Aus der JP-A-59-199670 ist ein Verfahren zur Herstellung eines granulierten Methionins bekannt, bei dem man Methionin und andere körnige organische oder anorganische Materialien als Pulver mischt und unter Zusatz von Lösungsmitteln granuliert.

Aufgabe der Erfindung ist, ein Verfahren bereitzustellen, das zu geformten, staubarmen, rieselfähigen Produkten führt.

Gegenstand der Erfindung sind rieselfähige Methionin-Extrudate, mit einem Schüttgewicht zwischen 300 und 850 kg/m³ insbesondere 600 bis 800 kg/m³, und einem Kornspektrum zwischen 63 µm und 5000 µm, insbesondere 500 µm bis 1500 µm. Sie enthalten Methionin in einer Menge von 60 bis 98 Gew.-%, bevorzugt 85 bis 95 Gew.-%. An den Extrudaten haftet von 0,1 bis 10 Gew.-% bezogen auf das Methionin an gelöstem Methionin und Kaliumsalze, und sie enthalten organische Nebenprodukte aus dem Methionin-Herstellprozess. Formlinge können nach dem aus dem Stand der Technik bekannten Verfahren hergestellt werden.

In einer bevorzugten Form enthalten die Extrudate, auch Fremdsubstanzen, die als Bindemittel wirken.

Diese sind organischer oder anorganischer Natur und geeignet als Zusätze bei der Verwendung der erfindungsgemäßen Produkte als Futtermittel.
In einer besonders geeigneten Ausführungsform stammen diese Verbindungen aus dem Herstellprozeß des Methionins und sind gegegebenenfalls als Nebenprodukte im Hauptprodukt mit enthalten, ohne daß sie zur Reinigung des Hauptprodukts vorher abgetrennt werden mußten.

D,L-Methionin oder ein Salz des D,L-Methionins wird ausgehend von den Komponenten 3-Methylmercaptopropionaldehyd, Cyanwasserstoff, Ammoniak und Kohlendioxid bzw. 5-(2-Methylmercaptoethyl)-hydantoin hergestellt.

Die Syntheseschritte lassen sich durch folgende Reaktionsgleichungen veranschaulichen:
5-(2-Methylmercapto)-hydantoinbildung: 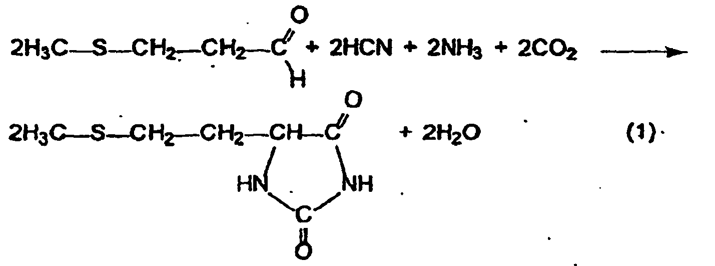
Salzbildung des D,L-Methionins: 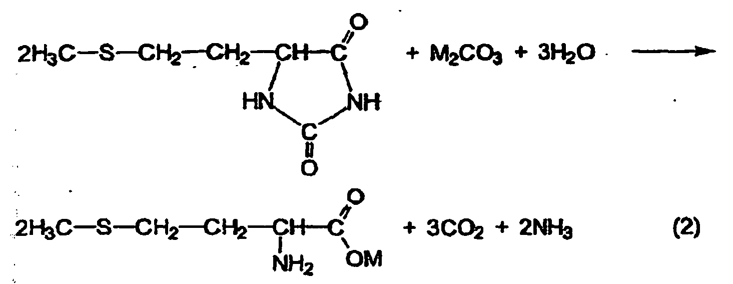
D,L-Methionin-Freisetzung: 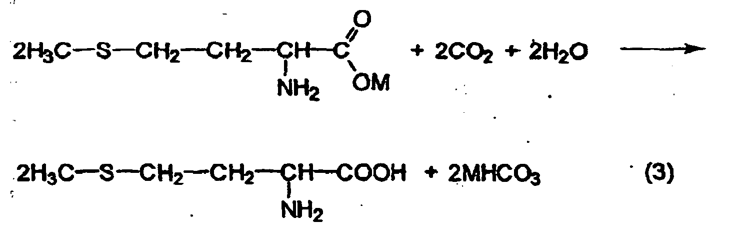
M steht für Alkali, Erdalkali, Ammonium, insbesondere Kalium.

Verfahren dieses Typs werden beispielsweise in der DE-PS 1906405 beschrieben.

Es ist für die Verformung von Vorteil, das aus dem Herstellprozeß abgetrennte Methionin mit Wasser oder mit einer dem Herstellprozeß entnommenen Mutterlauge zu waschen.
Dem Feststoff haften erfindungsgemäß noch Mengen im Bereich von 0,1 bis 10 Gew.-%, bezogen auf das Methionin an gelöstem Methionin und Kaliumsalzen, insbesondere Kaliumcarbonat und/oder Kaliumhydrogencarbonat an.

Ein Restfeuchtebereich von 8 bis 40 Gew.-%, insbesondere 12 bis 25 Gew.-%, bezogen auf die Gesamtmasse, hat sich als für die Formgebung vorteilhaft erwiesen.

In einer bevorzugten Form enthalten die Extrudate auch als Bindemittel, Kaliumcarbonat und/oder Kaliumhydrogencarbonat in einer Menge von >0 bis 20 Gew.-%, bezogen auf Methionin.

Zur Verbesserung des Formgebungsverfahrens werden auch organische Bindemittel zusätzlich, einzeln oder gemeinsam eingesetzt.

Dazu gehören gelöstes Methionin und weitere Stoffe, wie Methylcellulose, Ethylcellulose, Carboxymethylcellulose, Carboxyethylcellulose, Carboxypropylcellulose, Hydroxypropylcellulose, Hydroxypropylmethylcellulose, Celluloseacetatdibutylaminoacetat u. a. Cellulosederivate, Stärke, Hydroxypropylstärke, Zucker, Dextrin, Gelatine, weitere natürliche hochmolekulare Stoffe, Polyvinylalkohol, Polyvinylpyrrolidon, Polyvinylacetaldibutylaminoacetat, wobei man einen einzelnen oder mehrere dieser Stoffe nebeneinander verwenden und dadurch die Formgebung verbessern kann. Die Bindemittel werden bevorzugt in Form wäßriger Lösungen eingesetzt.

Zur Verbesserung der Handhabbarkeit der herzustellenden Formlinge ist es empfehlenswert, das Methionin in Gegenwart von Zuschlagstoffen auf silikatischer Basis zu verformen.

Zu diesen gehören hydrophile und hydrophobe Kieselsäuren pyrogener Natur oder durch Fällung hergestellte von 5 bis 300 m²/g, vorzugsweise sprühgetrocknete Kieselsäuren. Verwendbar sind auch feinteilige Zeolithe z. B. des Typs A oder Bentonite.

Die Menge dieser sowie weiterer Zuschlagstoffe beläuft sich auf 0,1 bis 10 Gew.-%, bevorzugt 0,1 bis 5 Gew.-%, bezogen auf das Methionin. Zu diesen Zuschlagstoffen gehören neben den silikatischen Verbindungen naturgemäß bevorzugt Substanzen, die im Futtermittelbereich zugelassen sind, insbesondere Fettsäuren und deren Salze, bevorzugt Alkali- oder Erdalkalisalze.
Zu den Fettsäuren gehören insbesondere Stearinsäure und Palmitinsäure bzw. Mischungen von C₁₆ bis C₁₈-Kohlenstoffatomen enthaltenden Fettsäuren oder deren obengenannten Salzen.
Bindemittel, Zuschlagstoffe und auch futterwirksame Verbindungen werden der Suspension des Methionins entweder fest oder gegebenenfalls in Form einer wäßrigen Lösung oder dem abgetrennten Methionin vor der Extrusion zugesetzt.

Zu den futterwirksamen Verbindungen gehören weitere Aminosäuren, wie Lysin, Threonin oder Alanin oder deren Salze,
N-Acylaminosäuren, N-Hydroxymethylmethionincalciumsalze, 2-Hydroxy-4-methylmercaptobuttersäure und andere Aminosäure-Hydroxyanaloge, Fischmehl, Kasein, weitere Proteine, Vitamin A, Vitamin A-Acetat, Vitamin A-Palmitat, Vitamin D3, Vitamin E, Nicotinsäure, Nicotinsäureamid, Pantothensäurecalcium, β-Carotin.

Diese Verbindungen werden in der Summe in einer Menge von 0 bis 40 Gew.-%, insbesondere 1 bis 20 Gew.-%, bezogen auf Methionin, eingesetzt.
Die erfindungsgemäß hergestellten Extrudate eignen sich dann als komplexes Futtermittel für die Tierernährung.

Ein weiterer Gegenstand der Erfindung ist ein Verfahren zur Herstellung von rieselfähigen, Methionin-haltigen Extrudaten dadurch gekennzeichnet, daß man im Anschluss an die Herstellung des Methionins aus den Komponenten 3-Methylmercaptopropionaldehyd, Cyanwasserstoff, Ammoniak und Kohlendioxid bzw. 5-(2-Methylmercaptoethyl)-hydantoin aus der Kaliumcarbonat/hydrogencarbonat und organischen Nebenprodukte enthaltenden wäßrigen Methioninsuspension das Wasser bis auf 8 bis 40 Gew.-%, bezogen auf die Gesamtmenge, abtrennt, den so erhaltenen Feststoff extrudiert und anschließend trocknet.
Insbesondere eine Restfeuchte von 12 bis 25 Gew.% hat sich als vorteilhaft für die Formgebung erwiesen.
Dies gilt vor allem dann, wenn man das Methionin im Anschluß an den Herstellprozeß verarbeitet und die Suspension Gehalte an Kaliumcarbonat/- Hydrogencarbonat und organischen Nebenprodukten aufweist.

Als besonders geeignet für die Verformung hat sich die Verwendung eines Zweischneckenextruders herausgestellt. Der Feststoff wird dann im allgemeinen bei Extrusionsdrücken bis zu 60 bar bei Temperaturen von 20 bis 120 ° C durch eine Extrusionsmatrize gepresst.
Auf diesem Wege werden Extrudate mit Extrudatdurchmessern zwischen 500 µm und 5000 µm, vorzugsweise 800 bis 1000 µm erzeugt.
Die Extrudatlänge kann entweder durch mechanisches Abschneiden eingestellt werden, oder aber das Extrudat zerfällt bei geeigneter Wahl der Verfahrensparameter beim Austritt aus der Matrize. Als weitere geeignete Apparaturen bzw. Verfahren zur Herstellung von Formlingen seien Einschneckenextruder, Ringmatrizenpressen oder Kollerwerke genannt.

Erfolgt die Verformung bei erhöhter Produkttemperatur, so ergibt sich eine zusätzliche Verdunstung von Wasser, die den abschließenden Trocknungsschritt deutlich entlastet.

In einigen Fällen ist es vorteilhaft, die erhaltenen Formlinge vor der endgültigen Trocknung in einem sogenannten "Spheronizer" zu behandeln, um eine Rundung der Teilchen zu erzielen.

Der abschließende gegebenenfalls unter Vakuum erfolgende Schritt der Abtrocknung der noch vorhandenen Restfeuchte aus den Formlingen muß zumindest zu Beginn mechanisch sehr schonend vorgenommen werden, um eine Beschädigung oder Zerstörung der Formlinge zu verhindern.

Bewährt hat sich eine Kombination von Trocknungsband zum Antrocknen der Formlinge, insbesondere der Extrudate, und dem abschließenden Endtrocknen in einem Tellertrockner. Die Produkttemperaturen liegen dabei im Bereich zwischen 50 und 140 ° C, bevorzugt bei 90 bis 110 ° C.

Insbesondere nach diesem Trocknungsverfahren weisen die Formlinge die gewünschte hohe mechanische Festigkeit auf. Diese erlaubt die einfache Handhabung des Produkts. Die Schüttdichte liegt dann im allgemeinen bei 600 bis 800 kg/m³.

Der Staubgehalt liegt im allgemeinen bei <1 %, gemessen nach der Methode von Dr. Groschopp.

### Beispiele

### Beispiel 1

Es wird Methionin mit einer Restfeuchte von 25 Gew.-%, einem K2C03-Gehalt von 1,7% und einem KHCO3-Gehalt von 2,8 % eingesetzt. Dieses wird mit 10 kg/h einem Zweischneckenextruder zugeführt. Über eine zweite Dosierung werden 120 g/h Tylose in den Einzug zugegeben. Der Extruder wird auf 110 ° C aufgeheizt, das Methionin-Tylose-Gemisch wird bei einem Druck von 15 bar über eine Düse mit 150 0,8-mm-Bohrungen extrudiert. Die Granulierung erfolgt durch ein unmittelbar auf der Düse rotierendes Messer. Das Granulat besitzt eine Austrittsfeuchte von 23 Gew.-%, wird auf einem Trocknungsband vor- und auf einem Tellertrockner endgetrocknet. Das Kornspektrum beträgt 400 bis 1000 µm. Der Methioningehalt des Endproduktes ist 88 Gew.-%. Der Staubgehalt ist <0,5 % und die Partikeln weisen eine gute Festigkeit auf.

### Beispiel 2

Das Beispiel 1 wird wiederholt mit dem Unterschied, daß mit einem Düsenkopf mit 80 1,2-mm-Bohrungen bei einem Druck von 13 bar extrudiert wird. Der Durchsatz beträgt 12 kg/h. Das Granulat besitzt eine Austrittsfeuchte von 23 %. Das Kornspektrum liegt bei 600 bis 1500 µm.

### Beispiel 3

Methionin mit einer Restfeuchte von 32 Gew.-%, einem K2CO3-Gehalt von 2,0 Gew.-% und einem KHCO3-Gehalt von 3,2 Gew.-% wird in einem Kontakttrockner auf eine Restfeuchte von 16 Gew.-% getrocknet. Dieses Produkt wird anschließend ohne weitere Zusätze in einem Zweischneckenextruder bei einer Temperatur von 30 ° C und einem Durchsatz von 15 kg/h über eine Düse mit 0,8-mm-Bohrungen extrudiert. Nach einer Messergranulation erfolgt anschließend eine Partikelrundung auf einem Spheronizer. Die Endtrocknung wird in einem Tellertrockner durchgeführt. Diese Vorgehensweise führt zu sphärischen Partikeln mit einem Kornspektrum von 300 bis 1000 µm. Der Staubgehalt ist <0,5 %.

### Beispiel 4

Das Beispiel 3 wird wiederholt mit dem Unterschied, daß statt eines Extruders eine Ringmatrizenpresse zur Formgebung eingesetzt wird. Das Kornspektrum liegt bei 400 bis 1200 µm. Der Staubgehalt ist <0,5 %.

## Patentansprüche

1. Rieselfähige Methionin-haltige Formlinge mit einem Schüttgewicht zwischen 300 und 850 kg/m³ und einem Kornspektrum zwischen 63 µm und 5000 µm, wobei das Methionin hergestellt wird aus 3-Methylmercaptopropionaldehyd, Cyanwasserstoff, Ammoniak und Kohlendioxid bzw. 5-(2-Methylmercaptoethyl)-hydantoin, **dadurch gekennzeichnet, dass**
a) an den Formlingen von 0,1 bis 10 Gew.-%, bezogen auf das Methionin, an gelöstem Methionin und Kaliumsalzen haftet,
b) die Formlinge einen Methioningehalt von 60 bis 98 Gew.-%, bezogen auf das Gesamtgewicht, haben,
c) organische Nebenprodukte aus dem Methionin-Herstellprozess enthalten, und
d) Extrudate sind.

2. Formlinge gemäss Anspruch 1, mit einem Methioningehalt von 85 bis 95 Gew.-%.

3. Formlinge gemäss den Ansprüchen 1 oder 2, die ein organisches und/oder anorganisches Bindemittel enthalten.

4. Formlinge gemäss Anspruch 3, die als Bindemittel Kaliumcarbonat oder Kaliumhydrogencarbonat, einzeln oder gemeinsam, in einer Menge von > 0 bis 20 Gew.-% enthalten.

5. Formlinge gemäss Anspruch 4, bei denen das Bindemittel aus der Mutterlauge der Methioninherstellung stammt.

6. Formlinge gemäss einem oder mehreren der vorhergehenden Ansprüche mit einem zusätzlichen Gehalt an organischen Bindemitteln, zu denen gehören gelöstes Methionin, Methylcellulose, Ethylcellulose, Carboxymethylcellulose, Carboxyethylcellulose, Carboxypropyl-cellulose, Hydroxypropylcellulose, Hydroxypropyl-methylcellulose, Celluloseacetatdibutylaminoacetat u.a. Cellulosederivate, Stärke, Hydroxypropylstärke, Zucker, Dextrin, Gelatine, Polyvinylalkohol, Polyvinylpyrrolidon, Polyvinylacetaldibutylaminoacetat einzeln oder im Gemisch miteinander.

7. Formlinge gemäss einem oder mehreren der vorhergehenden Ansprüche, die hydrophile und/oder hydrophobe Kieselsäuren und/oder Zuschlagstoffe auf silikatischer Basis enthalten.

8. Formlinge gemäss einem oder mehreren der vorhergehenden Ansprüche, die weitere futterwirksame Verbindungen wie Lysin, Threonin oder Alanin oder deren Salze, N-Acylamino-säuren, N-Hydroxymethylmethionincalciumsalze, 2-Hydroxy-4-methylmercaptobuttersäure und andere Aminosäure-Hydroxyanaloge, Fischmehl, Kasein, weitere Proteine, Vitamin A, Vitamin A-Acetat, Vitamin A-Palmitat, Vitamin D3, Vitamin E, Nicotinsäure, Nicotinsäureamid, Pantothensäurecalcium, β-Carotin, einzeln oder gemeinsam enthalten.

9. Verfahren zur Herstellung von rieselfähigen Methioninhaltigen Formlingen gemäss Anspruch 1, **dadurch gekennzeichnet , dass** man im Anschluss an die Herstellung des Methionins aus den Komponenten 3-Methylmercaptopropionaldehyd, Cyanwasserstoff, Ammoniak und Kohlendioxid bzw. 5-(2-Methylmercaptoethyl)hydantoin das Wasser aus der Kaliumcarbonat/hydrogencarbonat und organischen Nebenprodukte enthaltenden Methioninsuspension bis auf 8 bis 40 Gew.-%, bezogen auf die Gesamtmasse abtrennt, den so erhaltenen Feststoff einem formgebenden . Extrusionsverfahren unterwirft, und anschließend trocknet.

10. Verfahren gemäß Anspruch 9, **dadurch gekennzeichnet, daß** man das aus dem Herstellprozeß abgetrennte Methionin vor der Formgebung mit Wasser, wäscht.

11. Verfahren gemäss Anspruch 10, **dadurch gekennzeichnet, dass** man das aus dem Herstellprozeß abgetrennte Methionin vor der Formgebung mit einer dem Herstellprozeß entnommenen Mutterlauge wäscht.

12. Verfahren gemäss den Ansprüchen 9 oder 10, **dadurch gekennzeichnet, dass** man dem aus dem Herstellprozeß abgetrennten Methionin vor der Formgebung organische und/oder anorganische Bindemittel zusetzt.

13. Verfahren gemäss einem oder mehreren der Ansprüche 9-12, **dadurch gekennzeichnet, dass** man dem Methionin vor der Formgebung einen silikatischen Füllstoff zusetzt.

14. Verfahren gemäss Anspruch 13, **dadurch gekennzeichnet , dass** man dem Methionin vor der Formgebung eine hydrophile oder hydrophobe Kieselsäure zusetzt.

15. Verfahren gemäss einem oder mehreren der Ansprüche 9-14, **dadurch gekennzeichnet, dass** man dem Methionin vor der Formgebung eine oder mehrere weitere futterwirksame Verbindung zusetzt.

16. Verfahren gemäss einem oder mehreren der Ansprüche 9-14, **dadurch gekennzeichnet , dass** man das wasserhaltige Methionin bei einer Temperatur von 20 bis 120°C extrudiert, gegebenenfalls spheronisiert und anschließend trocknet.

17. Verfahren gemäss Anspruch 16, **dadurch gekennzeichnet, dass** das wasserhaltige Methionin in einem Zweischneckenextruder extrudiert wird.

18. Verfahren gemäss einem oder mehreren der Ansprüche 9-17, **dadurch gekennzeichnet, dass** man die Extrudate gegebenenfalls auf einem Trocknungsband und gegebenenfalls zusätzlich auf einem Tellertrockner bei Temperaturen von 50 bis 140°C gegebenenfalls unter Vakuum trocknet.

## Claims

1. Pourable methionine-containing shaped bodies with a bulk density of between 300 and 850 kg/m³ and a particle spectrum of between 63 µm and 5000 µm, wherein the methionine is prepared from 3-methylmercaptopropionaldehyde, hydrogen cyanide, ammonia and carbon dioxide or 5-(2-methylmercaptoethyl)-hydantoin,
**characterised in that**
a) 0.1 to 10 wt.%, based on the methionine, of dissolved methionine and potassium salts adheres to the shaped bodies,
b) the shaped bodies have a methionine content of 60 to 98 wt.%, based on the total weight,
c) they contain organic secondary products from the methionine production process, and
c) they are extrudates.

2. Shaped bodies according to claim 1, having a methionine content of 85 to 95 wt.%.

3. Shaped bodies according to claims 1 or 2, containing an organic and/or inorganic binder.

4. Shaped bodies according to claim 3, containing as binder potassium carbonate or potassium hydrogen carbonate, individually or together, in a quantity of > 0 to 20 wt.%.

5. Shaped bodies according to claim 4, in which the binder comes from the mother liquor of the methionine preparation.

6. Shaped bodies according to one or more of the preceding claims, additionally containing organic binders, which include dissolved methionine, methyl cellulose, ethyl cellulose, carboxymethyl cellulose, carboxyethyl cellulose, carboxypropyl cellulose, hydroxypropyl cellulose, hydroxypropyl methyl cellulose, cellulose acetate dibutylaminoacetate and other cellulose derivatives, starch, hydroxypropyl starch, sugar, dextrin, gelatin, polyvinyl alcohol, polyvinylpyrrolidone, polyvinylacetal dibutylaminoacetate, individually or in admixture with one another.

7. Shaped bodies according to one or more of the preceding claims, containing hydrophilic and/or hydrophobic silicas and/or silicate-based additives.

8. Shaped bodies according to one or more of the preceding claims, containing other feed-effective compounds such as lysine, threonine or alanine or their salts, N-acylamino acids, N-hydroxymethylmethionine calcium salts, 2-hydroxy-4-methylmercaptobutyric acid and other amino acid hydroxy analogues, fish meal, casein, other proteins, vitamin A, vitamin A acetate, vitamin A palmitate, vitamin D3, vitamin E, nicotinic acid, nicotinamide, pantothenic acid calcium, β-carotene, individually or together.

9. Process for the production of pourable methionine-containing shaped bodies according to claim 1, **characterised in that**, following the production of the methionine from the components 3-methylmercaptopropionaldehyde, hydrogen cyanide, ammonia and carbon dioxide or 5-(2-methylmercaptoethyl)-hydantoin, the water is removed from the methionine suspension containing potassium carbonate/potassium hydrogen carbonate and organic secondary products down to 8 to 40 wt.%, based on the total weight, and the solid so obtained is subjected to a shaping extrusion process and is subsequently dried.

10. Process according to claim 9, **characterised in that**, prior to shaping, the methionine separated from the preparation process is washed with water.

11. Process according to claim 10, **characterised in that**, prior to shaping, the methionine separated from the preparation process is washed with a mother liquor taken from the preparation process.

12. Process according to claims 9 or 10, **characterised in that**, prior to shaping, organic and/or inorganic binders are added to the methionine separated from the preparation process.

13. Process according to one or more of claims 9 to 12, **characterised in that** a siliceous filler is added to the methionine prior to shaping.

14. Process according to claim 13, **characterised in that** a hydrophilic or hydrophobic silica is added to the methionine prior to shaping.

15. Process according to one or more of claims 9 to 14, **characterised in that** one or more other feed-effective compounds are added to the methionine prior to shaping.

16. Process according to one or more of claims 9 to 14, **characterised in that** the water-containing methionine is extruded at a temperature of 20 to 120°C, optionally spheronised and subsequently dried.

17. Process according to claim 16, **characterised in that** the water-containing methionine is extruded in a twinscrew extruder.

18. Process according to one or more of claims 9 to 17, **characterised in that** the extrudates are optionally dried on a drying belt and optionally additionally on a disk dryer at temperatures of 50 to 140°C, optionally *in vacuo.*

## Revendications

1. Pièces façonnées pouvant s'écouler, contenant de la méthionine, présentant une densité apparente entre 300 et 850 kg/m² et un spectre granulométrique entre 63 µm et 5000 µm, dont la méthionine est préparée en partant des composants 3-méthylmercaptopropinoladéhyde, acide cyanhydrique, ammoniac et dioxyde de carbone ou 5-(2-méthylmercaptoéthyl)hydantoïne, **caractérisées en ce que**
a) 0,1 à 10 % en poids, par rapport à la méthionine, de méthionine dissoute et de sels de potassium adhère aux pièces façonnées,
b) les pièces façonnées présentent une teneur en méthionine de 60 à 98% en poids par rapport au poids total,
c) les pièces façonnés comprennent des produits secondaires organiques obtenus du processus de production de la méthionine, et
d) les pièces façonnées sont des pièces extrudées.

2. Pièces façonnées selon la revendication 1, présentant une teneur en méthionine de 85 à 95% en poids.

3. Pièces façonnées selon les revendications 1 ou 2, qui contiennent un liant organique et/ou inorganique.

4. Pièces façonnées selon la revendication 3, qui contiennent du carbonate de potassium ou de l'hydrogénocarbonate de potassium, seuls ou en mélange, en une quantité de > 0 à 20% en poids, comme liants.

5. Pièces façonnées selon la revendication 4, **caractérisées en ce que** le liant provient de la lessive-mère de la préparation de la méthionine.

6. Pièces façonnées selon l'une ou plusieurs des revendications précédentes, présentant une teneur supplémentaire en liants organiques, auxquels appartiennent la méthionine dissoute, la méthylcellulose, l'éthylcellulose, la carboxyméthylcellulose, la carboxyéthylcellulose, la carboxypropylcellulose, l'hydroxypropylcellulose, l'hydroxypropylméthylcellulose, l'acétatedibutylaminoacétate de cellulose et d'autres dérivés de la cellulose, les amidons, les hydroxypropylamidons, le sucre, la dextrine, la gélatine, le poly(alcool vinylique), la polyvinylpyrrolidone, le poly(acétaldibutylaminoacétate de vinyle), seuls ou en mélange les uns avec les autres.

7. Pièces façonnées selon l'une ou plusieurs des revendications précédentes, qui contiennent des silices hydrophiles et/ou hydrophobes et/ou des adjuvants à base de silicates.

8. Pièces façonnées selon l'une ou plusieurs des revendications précédentes, qui contiennent d'autres composés actifs comme fourrages, tels que la lysine, la thréonine ou l'alanine ou leurs sels, les N-acylaminoacides, les sels calciques de la N-hydroxyméthylméthionine, l'acide 2-hydroxy-4-méthylmercaptobutyrique et d'autres analogues hydroxy d'aminoacides, la farine de poissons, la caséine, d'autres protéines, la vitamine A, l'acétate de la vitamine A, le palmitate de vitamine A, la vitamine D3, la vitamine 3, l'acide nicotinique, l'amide de l'acide nicotinique, le sel calcique de l'acide pantothénique, le β-carotène, seuls ou ensemble.

9. Procédé pour la préparation de pièces façonnées pouvant s'écouler contenant de la méthionine selon la revendication 1, **caractérisé en ce qu'**on sépare l'eau de la suspension de méthionine, contenant de la carbonate de potassium/carbonate de hydrogène et des produits secondaires organiques, à la suite de la production de la méthionine des composants 3-méthylmercaptopropinoladéhyde, acide cyanhydrique, ammoniac et dioxyde de carbone ou 5-(2-méthylmercaptoéthyl)hydantoïne jusqu'à 8 à 40 % en poids par rapport à la masse totale, qu'on soumet le solide ainsi obtenu à un procédé d'extrusion de façonnage et qu'on sèche ensuite.

10. Procédé selon la revendication 9, **caractérisé en ce qu'**on lave la méthionine séparée du processus de préparation avec de l'eau avant le façonnage.

11. Procédé selon la revendication 10, **caractérisé en ce qu'**on lave la méthionine séparée du processus de préparation avec de la lessive-mère prélevée du processus de préparation avant le façonnage.

12. Procédé selon les revendications 9 ou 10, **caractérisé en ce qu'**on ajoute à la méthionine séparée du processus de préparation des liants organiques et/ou inorganiques avant le façonnage.

13. Procédé selon l'une ou plusieurs des revendications 9 à 12, **caractérisé en ce qu'**on ajoute une charge silicatée à la méthionine avant le façonnage.

14. Procédé selon la revendication 13, **caractérisé en ce qu'**on ajoute à la méthionine une silice hydrophile ou hydrophobe avant le façonnage.

15. Procédé selon l'une ou plusieurs des revendications 9-14, **caractérisé en ce qu'**on ajoute à la méthionine un ou plusieurs autres composés actifs comme fourrages avant le façonnage.

16. Procédé selon l'une ou plusieurs des revendications 9-14, **caractérisé en ce qu'**on extrude la méthionine contenant de l'eau à une température de 20 à 120°C, qu'on la transforme le cas échéant en sphères et qu'on la sèche ensuite.

17. Procédé selon la revendication 16, **caractérisé en ce que** la méthionine contenant de l'eau est extrudée dans une extrudeuse à deux vis sans fin.

18. Procédé selon l'une ou plusieurs des revendications 9 à 17, **caractérisé en ce qu'**on sèche les pièces extrudées le cas échéant sur une bande de séchage et, de plus, le cas échéant, sur un sécheur à assiette à des températures de 50 à 140°C, le cas échéant sous vide.
